(19) Europäisches Patentamt
European Patent Office
Office européen des brevets

(11) EP 0 591 058 B1

(12) **FASCICULE DE BREVET EUROPEEN**

(45) Date de publication et mention de la délivrance du brevet: **03.06.1998 Bulletin 1998/23**

(51) Int. Cl.$^6$: **C07D 471/04**, A61K 31/435 // (C07D471/04, 221:00, 209:00)

(21) Numéro de dépôt: **93402395.3**

(22) Date de dépôt: **01.10.1993**

(54) **Dérivés d'Ellipticine à activité antitumorale**

Ellipticinderivate mit Antitumorwirkung

Ellipticine derivatives with antitumor activity

(84) Etats contractants désignés:
**AT BE CH DE DK ES FR GB GR IE IT LI LU NL PT SE**

(30) Priorité: **02.10.1992 FR 9211672**

(43) Date de publication de la demande:
**06.04.1994 Bulletin 1994/14**

(73) Titulaire: **ADIR ET COMPAGNIE**
**92415 Courbevoie Cédex (FR)**

(72) Inventeurs:
- **Bisagni, E.**
  **F-91400 Orsay (FR)**
- **Jasztold-Howorko, R.**
  **PL-21 53-326 Wroclaw (PL)**
- **Atassi, G.**
  **F-92400 Saint-Cloud (FR)**
- **Pierre, A.**
  **F-78160 Marly-le-Roi (FR)**

(56) Documents cités:
- **JOURNAL OF HETEROCYCLIC CHEMISTRY vol. 19 , 1982 , PROVO US pages 1275 - 1280 F.D. POPP ET AL. 'Synthesis of potential antineoplastic agents. XXVII. (Reissert compound studies. XLIV.). The ellipticine Reissert compound as an intermediate in the synthesies of ellipticine analogs'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 591 058 B1

## Description

La présente invention concerne de nouveaux composés d'ellipticine, leur procédé de préparation et les compositions pharmaceutiques qui les contiennent. Les composés de la présente invention trouvent une utilisation thérapeutique intéressante grâce à leur activité antitumorale.

Des dérivés de la famille de l'ellipticine sont déjà connus pour leurs propriétés anticancéreuses. On peut citer à titre d'exemple le brevet EP-A-42348 où des composés de l'olivacine ont été étudiés. Les brevets EP-A-393575 et EP-A-10029 décrivent des dérivés des pyrido[4,3-b]carbazoles ou ellipticines.

Les besoins de la thérapeutique exigent le développement constant de nouveaux anti-cancéreux dans le but d'obtenir des molécules à la fois plus actives et mieux tolérées.

La présente invention concerne des dérivés de l'ellipticine présentant une originalité par rapport aux composés décrits dans l'art antérieur. En effet, l'intensité des propriétés pharmacologiques, c'est-à-dire l'activité antitumorale des produits de l'invention a été optimisée en greffant sur le 6*H*-pyrido[4,3-b]carbazole un groupement aminoalkyl-aminocarbonyle en position 1.

Par ailleurs, les composés de la présente invention se distinguent par leur plus grande cytotoxicité in vitro ainsi que leur meilleure activité in vivo par rapport aux produits de référence.

Plus particulièrement, la présente invention a pour objet des molécules de formule générale (I) :

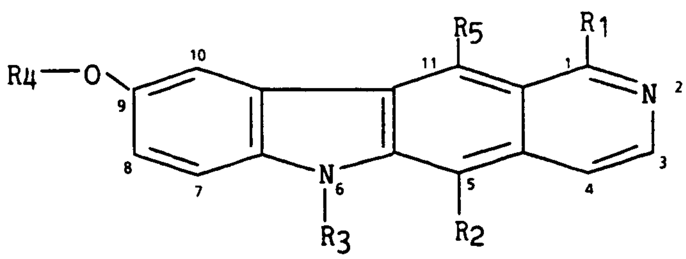

(I)

dans laquelle :

$R_1$ représente le radical

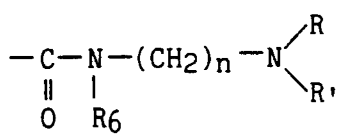

dans lequel :

- n est un entier compris entre 1 et 6,
- R, R' et $R_6$ identiques ou différents sont choisis indépendamment les uns des autres parmi l'hydrogène et un radical alkyle linéaire ou ramifié contenant 1 à 6 atomes de carbone, éventuellement substitués par un ou plusieurs groupements hydroxy,
- ou bien R et R' forment ensemble avec l'atome d'azote auquel ils sont rattachés un hétérocycle contenant éventuellement un second hétéroatome, lequel hétérocycle peut être substitué par un ou plusieurs radicaux alkyles linéaires ou ramifiés contenant 1 à 6 atomes de carbone, et $R_6$ est tel que défini précédemment,
- ou bien R et $R_6$ forment ensemble un hétérocycle substitué ou non par un ou plusieurs radicaux alkyles linéaires ou ramifiés contenant 1 à 6 atomes de carbone, et R' est tel que défini précédemment,
- $R_2$ représente un radical alkyle linéaire ou ramifié contenant 1 à 6 atomes de carbone,
- $R_3$, $R_4$ et $R_5$ identiques ou différents sont choisis indépendamment les uns des autres parmi l'hydrogène et un radical alkyle linéaire ou ramifié contenant 1 à 6 atomes de carbone,

leurs isomères optiques ainsi que leurs éventuels N-oxydes et sels d'addition à un acide ou à une base, pharmaceutiquement acceptables.

Parmi les hétérocycles formés par l'ensemble des radicaux R et R' et l'atome d'azote auxquels ils sont rattachés, on entend plus particulièrement le pyrrole, les pyrrolines, la pyrrolidine, l'imidazole, les imidazolines, l'imidazolidine, le

pyrazole, les pyrazolines, l'oxazole, les oxazolines, l'oxazolidine, la pyridine, la pipéridine, la pyridazine, la pirymidine, la pyrazine, la pipérazine, la morpholine et la thiomorpholine.

L'invention s'étend aussi au procédé de préparation des composés de formule (I) caractérisé par une première réaction biomimétique de Besselievre Husson (Tetrahedron, (1981), 37, 241-246) dans laquelle on fait réagir en milieu acide, par exemple dans l'acide acétique, un composé de formule (II) avec un composé de formule (III) :

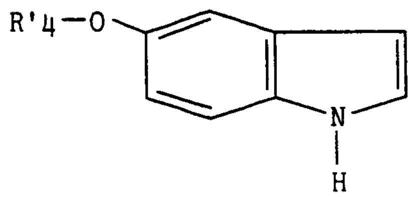

(II)

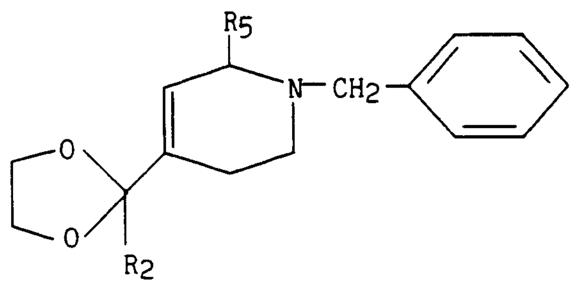

(III)

dans lesquelles $R_2$ et $R_5$ possèdent les mêmes significations que dans la formule (I) et $R'_4$ représente un radical alkyle linéaire ou ramifié contenant 1 à 6 atomes de carbone,
pour obtenir, après débenzylation, puis chauffage en présence de dialkyloxalate un composé de formule (IV)

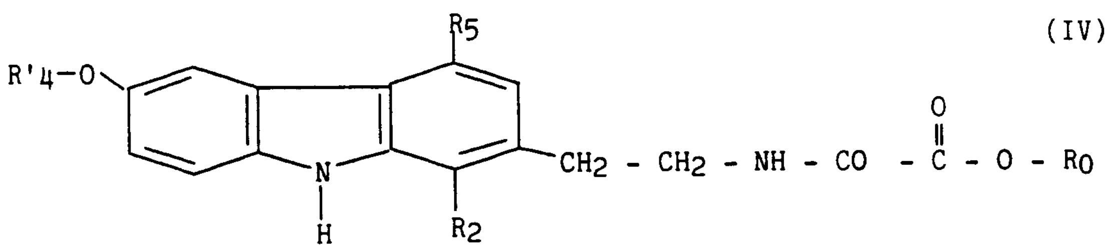

(IV)

dans laquelle $R_2$, $R'_4$ et $R_5$ possèdent les mêmes significations que précédemment et $R_0$ représente un groupement alkyle contenant de 1 à 5 atomes de carbone,
dont un chauffage à reflux en présence de $POCl_3$ dans le toluène permet la cyclisation, pour conduire après déshydrogénation sur charbon au palladium au composé de formule (Va) :

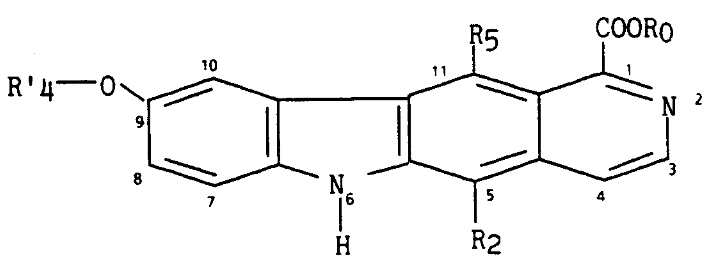

(Va)

dans laquelle $R_0$, $R_2$, $R'_4$ et $R_5$ ont les mêmes significations que précédemment,
dont on substitue, si on le désire, l'azote 6 du carbazole par traitement avec un carbonate de dialkyle, de formule

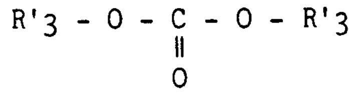

dans laquelle $R'_3$ représente un radical alkyle linéaire ou ramifié contenant 1 à 6 atomes de carbone, dans un solvant polaire, tel que la diméthylformamide, en présence d'un carbonate alcalin, tel que le carbonate de potassium, et d'un éther couronne, pour obtenir un composé de formule (Vb) :

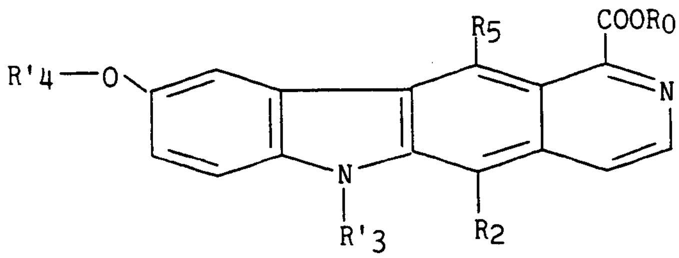

(Vb)

dans laquelle $R_2$, $R'_3$, $R'_4$, $R_5$ et $R_0$ possèdent les mêmes significations que précédemment, composés de formule (Va) et (Vb) formant l'ensemble des composés de formule (V) :

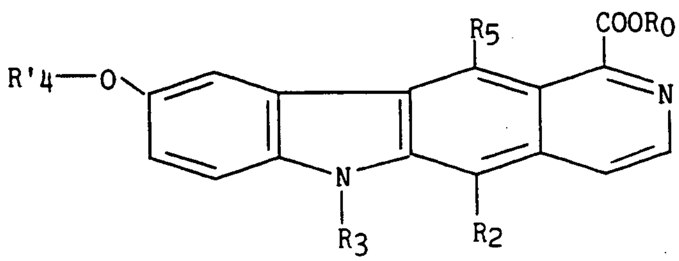

(V)

dans laquelle $R_2$, $R_3$, $R'_4$, $R_5$ et $R_0$ possèdent les mêmes significations que précédemment, composés de formule (V) que l'on soumet à une réaction de substitution par un composé de formule (VI) :

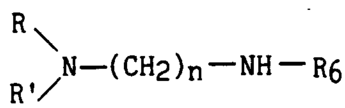

(VI)

dans laquelle R, R', $R_6$ et n possèdent les mêmes significations que dans la formule (I), pour obtenir les composés de formule (Ia) :

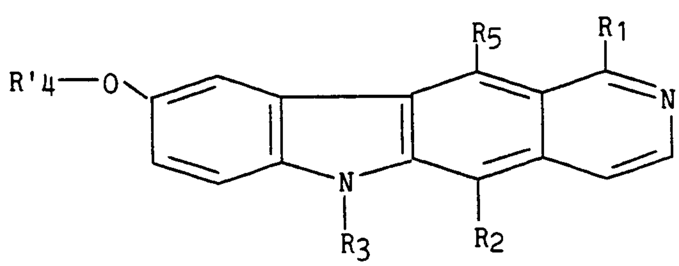

(Ia)

dans laquelle $R_1$, $R_2$, $R_3$, $R'_4$ et $R_5$ possèdent les mêmes significations que précédemment, composés de formule (Ia) que l'on peut déalkyler par ajout d'un trihalogénure de bore, tel que le tribromure de bore, pour donner les composés de formule (Ib) :

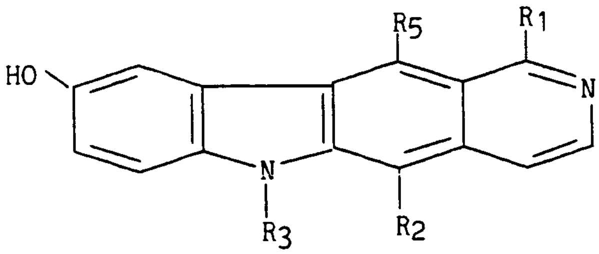

(Ib)

dans laquelle $R_1$, $R_2$, $R_3$ et $R_5$ ont les mêmes significations que précédemment, composés de formules (Ia) et (Ib) formant l'ensemble des composés de formule générale (I) que l'on purifie le cas échéant selon une technique classique de séparation et que l'on transforme, si nécessaire, en leurs éventuels N-oxydes et sels d'addition à un acide ou une base, pharmaceutiquement acceptables.

La réaction de déalkylation décrite pour la transformation du composé de formule (Ia) en composé de formule (Ib) peut également être effectuée directement sur l'ester de formule (V) décrit précédemment, de façon à obtenir le composé de formule (V') :

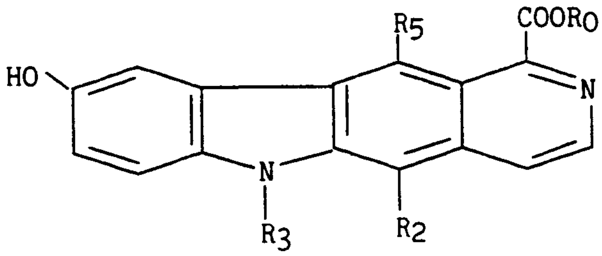

(V')

dans laquelle $R_2$, $R_3$, $R_5$ et $R_0$ sont tels que décrits précédemment, qui est ensuite traité par le composé de formule (VI) précédemment défini dans les mêmes conditions opératoires que celles définies pour le composé de formule (V), pour conduire au composé de formule (Ib) précédemment défini.

Les composés de formule (I) possèdent d'intéressantes propriétés pharmacologiques. Outre leur plus grande cytotoxicité in vitro, ils s'avèrent plus efficaces et au moins aussi actifs in vivo que les composés pris comme référence.

De plus, les composés décrits dans la présente invention sont actifs à des doses très faibles et donnent de nombreux survivants aux doses optimales. Ils présentent donc un index thérapeutique excellent.

Grâce à leur faculté de s'intercaler dans l'ADN et de provoquer des coupures en inhibant les topoisomérases, ils trouvent une utilisation en thérapeutique en tant qu'agents antitumoraux.

La présente invention a également pour objet les compositions pharmaceutiques contenant les produits de formule (I), leurs N-oxydes, leurs isomères optiques ou un de leurs sels d'addition à une base ou un acide, pharmaceutiquement acceptables, seul ou en combinaison avec un ou plusieurs excipients ou véhicules inertes, non toxiques.

Parmi les compositions pharmaceutiques selon l'invention, il sera cité plus particulièrement celles qui conviennent pour l'administration orale, parentérale, nasale, rectale, perlinguale, oculaire ou respiratoire, et notamment les comprimés simples ou dragéifiés, les comprimés sublinguaux, les sachets, les paquets, les gélules, les glossettes, les tablettes, les suppositoires, les crèmes, les pommades, les gels dermiques, les préparations injectables ou buvables, les aérosols, les gouttes oculaires ou nasales.

La posologie utile varie selon l'âge et le poids du patient, la voie d'administration, la nature de l'indication thérapeutique et des traitements éventuellement associés et s'échelonne entre 0,1 et 400 mg par jour, en une ou plusieurs administrations.

Les exemples suivants illustrent l'invention. Les produits de départ sont connus ou préparés à partir de modes opératoires connus.

**Préparation du 2-(β-Benzylamino)éthyl-6-méthoxy-1-méthylcarbazole**

Le 5-méthoxy indole et la 1-benzyl-4-(1,1-éthylènedioxyéthyl)-1,2,3,6-tétrahydropyridine (point d'ébullition : 184-188°C sous 10 mm de Hg, obtenu par réduction au borohydrure de sodium du chlorure de 1-benzyl-4-(1,1-éthylènedioxyéthyl)pyridinium dans le méthanol) sont chauffés à reflux pendant 66 heures dans l'acide acétique à 50 %. Le mélange est ensuite versé dans 2 litres d'eau. Après extraction par du dichlorométhane et évaporation du solvant, un

résidu huileux, qui est ensuite repris dans l'acétate d'éthyle est obtenu. Le solide progressivement formé est filtré et lavé avec le minimum nécessaire de dichlorométhane froid. Des cristaux d'acétate incolores sont obtenus. La base correspondante cristallise dans le toluène pour donner des cristaux incolores.

Point de fusion : 150°C

**Exemple 1: 1-(N,N-Diméthylaminoéthylaminocarbonyl)-5,6-diméthyl-9-hydroxy-6*H*-pyrido[4,3-b]carbazole**

Stade A : 1-Méthyl-2-[(β-éthoxalamido)éthyl]-6-méthoxycarbazole

35,6 g (88 mmol) de 2-[(β-benzylamino)éthyl]-6-méthoxy-1-méthyl carbazole (sous forme d'acétate) sont dissous dans 400 ml d'acide acétique auxquels on ajoute 10 g de charbon à 10 % de palladium. Le mélange est chauffé à 50°C et agité sous hydrogène à pression normale en maintenant la température jusqu'à ce que le volume d'hydrogène théorique soit consommé (10 heures). Le solvant est évaporé sous pression réduite ; le résidu obtenu est repris dans 200 ml d'eau. Après filtration et ajout d'ammoniaque, le solide est séché à l'air et recristallisé dans du toluène. 18,16 g de cristaux de couleur beige sont obtenus.

Rendement : 81,4 %
Point de fusion : 167-168°C

Le solide est chauffé à 100-110°C dans 70 ml d'oxalate de diéthyle pendant 1 heure et le mélange obtenu est évaporé à sec. Le résidu solide est repris dans du cyclohexane, filtré et recristallisé dans l'acétate d'éthyle pour donner 23,92 g du produit attendu.

Rendement : 94,6 %
Point de fusion : 144°C

| Analyse élémentaire : $C_{20}H_{22}N_2O_4$ | | | |
|---|---|---|---|
| | C % | H % | N % |
| calculé | 67,78 | 6,26 | 7,91 |
| trouvé | 67,96 | 6,35 | 8,21 |

Stade B : 1-(3,4-Dihydro-9-méthoxy-5-méthyl-6*H*-pyrido[4,3-b]carbazolyl) carboxylate d'éthyle

3,54 g (10 mmol) de l'amide obtenue à l'étape A sont dissous dans 300 ml de toluène bouillant et traités goutte à goutte par 30 ml de $POCl_3$. Le reflux est maintenu pendant 24 heures. Le résidu est concentré sous pression réduite puis repris dans 200 ml d'eau. La solution est filtrée et ajustée à pH 9-10 par ajout de carbonate de sodium. Le solide résultant est lavé à l'eau, séché et recristallisé dans l'acétate d'éthyle. 2,36 g de cristaux jaunes sont obtenus.

Rendement : 70 %
Point de fusion : 233-234°C

| Analyse élémentaire : $C_{20}H_{20}N_2O_3$ | | | |
|---|---|---|---|
| | C % | H % | N % |
| calculé | 71,41 | 5,99 | 8,33 |
| trouvé | 71,39 | 5,81 | 8,11 |

Stade C : 1-(9-Méthoxy-5-méthyl-6*H*-pyrido[4,3-b]carbazolyl) carboxylate d'éthyle

2,036 g (6 mmol) de l'ester obtenu au stade précédent sont portés à reflux dans 40 ml de diphényl éther en présence de 300 mg de charbon à 10 % de palladium pendant 10 minutes, ou dans la décaline, pendant 45 minutes, en présence de 200 mg de charbon à 10 % de Pd, ou dans le mésitylène pendant 5 heures en présence des mêmes quantités de l'agent de déshydrogénation. Le résidu est extrait par de l'acide chlorhydrique 1N, la solution aqueuse étant neutralisée par du bicarbonate de sodium. Le précipité obtenu est extrait au dichlorométhane. Après évaporation du solvant, le résidu solide restant est purifié par chromatographie sur colonne de gel de silice. L'élution par du dichlorométhane conduit à des traces de diphényl éther. Le produit attendu est élué par un mélange dichlorométhane/acétate d'éthyle, 98/2. Le produit est recristallisé dans de l'acétate d'éthyle. 1,17 g de cristaux jaunes sont obtenus.

Rendement : 58,3 %
Point de fusion : 255°C

| Analyse élémentaire : $C_{20}H_{18}N_2O_3$ | | | |
|---|---|---|---|
| | C % | H % | N % |
| calculé | 71,84 | 5,43 | 8,38 |
| trouvé | 71,75 | 5,54 | 8,45 |

Stade D : 1-(5,6-Diméthyl-9-méthoxy-6*H*-pyrido[4,3-b]carbazolyl) carboxylate d'éthyle

Un mélange constitué de 334 mg (1 mmol) de l'ester obtenu au stade précédent, de 250 mg de carbonate de potassium sec finement pulvérisé, de 5 ml de carbonate de diméthyle, de 1 ml de diméthylformamide et de 1 goutte d'éther couronne 18C6 est chauffé à reflux sous agitation pendant 8 heures. Après évaporation à sec, le résidu est repris à l'eau. Le solide obtenu est séché à l'air puis recristallisé dans du cyclohexane. 270 mg de cristaux jaunes sont obtenus.

Rendement : 77,5 %
Point de fusion : 162-164°C

| Analyse élémentaire : $C_{21}H_{20}N_2O_3$ | | | |
|---|---|---|---|
| | C % | H % | N % |
| calculé | 72,39 | 5,79 | 8,04 |
| trouvé | 72,16 | 5,91 | 8,03 |

Stade E : 1-(N,N-Diméthylaminoéthylaminocarbonyl)-5,6-diméthyl-9-méthoxy-6*H*-pyrido[4,3-b] carbazole

348 mg (1 mmol) de l'ester obtenu au stade précédent sont chauffés dans 5 ml de N,N-diméthyl-1,2-diaminoéthane pendant 18 heures. L'amine en excès est évaporée sous pression réduite. Le résidu obtenu est repris à l'eau ; le solide résultant est séché à l'air et recristallisé dans du cyclohexane. 234 mg de cristaux jaunes sont obtenus.

Rendement : 60 %
Point de fusion : 139°C

| Analyse élémentaire : $C_{23}H_{26}N_4O_2$ | | | |
|---|---|---|---|
| | C % | H % | N % |
| calculé | 70,74 | 6,71 | 14,35 |
| trouvé | 70,53 | 6,96 | 14,35 |

Stade F : 1-(N,N-Diméthylaminoéthylaminocarbonyl)-5,6-diméthyl-9-hydroxy-6*H*-pyrido[4,3-b]carbazole

390 mg (1 mmol) du composé obtenu au stade E sont dissous dans 40 ml de dichlorométhane sec sous atmosphère d'argon. La solution est refroidie à -70°C. On ajoute goutte à goutte 10 équivalents molaires de tribromure de bore, soit 10 ml d'une solution commerciale 1M dans le dichlorométhane. On laisse le mélange revenir à température ambiante (18 heures), puis on le verse dans 100 ml d'eau glacée. La solution est ajustée à pH basique par ajout de triéthylamine puis laissée sous agitation à température ambiante pendant 3 heures. Le produit est extrait au dichlorométhane et le solvant évaporé afin d'effectuer une chromatographie sur colonne d'alumine, (éluant dichlorométhane/éthanol, 95/5). Le produit final cristallise dans l'acétate d'éthyle qui est évaporé. 164 mg de cristaux jaunes sont obtenus.

Rendement : 43,6 %
Point de fusion : 256°C

| Analyse élémentaire : $C_{22}H_{24}N_4O_2$ | | | |
|---|---|---|---|
| | C % | H % | N % |
| calculé | 70,18 | 6,43 | 14,88 |
| trouvé | 69,79 | 6,69 | 14,49 |

**Exemple 2: 1-(N,N-Diméthylaminopropylaminocarbonyl)-5,6-diméthyl-9-hydroy-6*H*-pyrido[4,3-b]carbazole**

Ce composé est préparé selon le même protocole que celui décrit dans l'exemple 1, pour les stades A à D.

Stade E : 1-(N,N-Diméthylaminopropylaminocarbonyl)-5,6-diméthyl-9-méthoxy-6*H*-pyrido[4,3-b]carbazole

Le temps de chauffage de l'ester obtenu au stade E est ramené à 8 heures et la diamine utilisée est le N,N-diméthyl-1,3-diaminopropane. Le produit obtenu est extrait au dichlorométhane ; le solvant est ensuite évaporé et le résidu traité par un excès d'acide maléique dans l'acétone pour obtenir le sel correspondant.

Stade F : 1-(N,N-Diméthylaminopropylaminocarbonyl)-5,6-diméthyl-9-hydroxy-6*H*-pyrido[4,3-b]carbazole

404 mg (1 mmol) de l'amide obtenue au stade précédent sont dissous dans 40 ml de dichlorométhane sec sous atmosphère d'argon. La solution est refroidie à - 70°C. 10 équivalents molaires de tribromure de bore sont ajoutés goutte à goutte ; le mélange est laissé à température ambiante pendant 18 heures, puis versé dans 100 ml d'eau glacée. La solution est rendue basique par ajout de triéthylamine et agitée à température ambiante pendant 3 heures. Le produit est extrait au dichlorométhane l'évaporation du solvant fournit un résidu qui cristallise dans l'acétate d'éthyle.

Rendement : 76,8 %
Point de fusion : 198°C

| Analyse élémentaire : $C_{23}H_{26}N_4O_2$, 0,5 $H_2O$ | | | |
|---|---|---|---|
| | C % | H % | N % |
| calculé | 69,15 | 6,81 | 14,02 |
| trouvé | 69,55 | 6,80 | 13,94 |

**Exemple 3: 1-(N,N-Diméthylaminoéthylaminocarbonyl)-5,6-diméthyl-9-méthoxy-6*H*-pyrido[4,3-b]carbazole**

Ce composé est préparé selon le même protocole que celui décrit dans l'exemple 1 à l'exception du stade F.

Rendement : 60 %

Point de fusion : 139°C

| Analyse élémentaire : $C_{23}H_{26}N_4O_2$ | | | |
|---|---|---|---|
| | C % | H % | N % |
| calculé | 70,74 | 6,71 | 14,35 |
| trouvé | 70,53 | 6,96 | 14,35 |

**Exemple 4 : 1-(N,N-Diméthylaminopropylaminocarbonyl)-5,6-diméthyl-9-méthoxy-6*H*-pyrido[4,3-b]carbazole**

Ce composé est préparé selon le même protocole que celui décrit dans l'exemple 2 à l'exception du stade F.

Rendement : 92,5 %
Point de fusion : 139°C

| Analyse élémentaire : $C_{24}H_{28}N_4O_2$, $2C_4H_4O_4$ | | | |
|---|---|---|---|
| | C % | H % | N % |
| calculé | 60,37 | 5,70 | 8,80 |
| trouvé | 60,28 | 5,62 | 9,03 |

**Exemple 5: 1 (N,N-Diméthylaminopropylaminocarbonyl)-5-méthyl-9-méthoxy-6*H*-pyrido[4,3-b]carbazole**

Ce composé est préparé selon le même protocole que celui décrit dans l'exemple 1, à l'exception des stades D et F qui sont supprimés et des modifications suivantes au stade E :

- l'amine utilisée est le N,N-diméthyl-1,3-diaminopropane,
- le solide, après avoir été séché, est recristallisé dans l'acétate d'éthyle,

Rendement : 93 %
Point de fusion : 206°C

| Analyse élémentaire : $C_{23}H_{26}N_4O_2$, $H_2O$ | | | |
|---|---|---|---|
| | C % | H % | N % |
| calculé | 67,62 | 6,91 | 13,72 |
| trouvé | 67,60 | 6,72 | 14,05 |

**Exemple 6 : 1-(N,N-Diméthylaminoéthylaminocarbonyl)-5-méthyl-9-hydroxy-6*H*-pyrido[4,3-b]carbazole**

Ce composé est préparé selon le même protocole que celui décrit dans l'exemple 1 à l'exception du stade intermédiaire D et des modifications suivantes apportées aux stades E et F :

Stade E : -le temps de chauffage est ramené à 8 heures,
Stade F : -le composé obtenu après agitation pendant 3 heures à température ambiante étant insoluble dans le dichlorométhane, il est filtré et recristallisé dans l'acétate d'éthyle,

Rendement : 47 %
Point de fusion : > 270°C

| Analyse élémentaire : $C_{21}H_{22}N_4O_2$ | | | |
|---|---|---|---|
| | C % | H % | N % |
| calculé | 69,59 | 6,12 | 15,46 |
| trouvé | 69,40 | 6,14 | 15,21 |

**Exemple 7 : 1-(N,N-Diméthylaminopropylaminocarbonyl)-5-méthyl-9-hydroxy-6*H*-pyrido[4,3-b]carbazole**

Ce composé est préparé selon le même protocole que celui décrit dans l'exemple 1 à l'exception du stade intermédiaire D et des modifications suivantes :

Stade E : -l'amine utilisée est le N,N-diméthyl-1,3-diaminopropane, -le solide obtenu est séché à l'air et recristallisé dans l'acétate d'éthyle,

Stade F : -le produit obtenu après 3 heures d'agitation est filtré et recristallisé dans l'acétate d'éthyle,

Rendement : 56 %
Point de fusion : 218°C

| Analyse élémentaire : $C_{22}H_{24}N_4O_2$, 0,5$H_2O$ | | | |
|---|---|---|---|
| | C % | H % | N % |
| calculé | 68,57 | 6,49 | 14,55 |
| trouvé | 68,45 | 6,43 | 14,86 |

**Exemple 8: 1-(N,N-Diméthylaminoéthylaminocarbonyl)-5-méthyl-9-méthoxy-6*H*-pyrido[4,3-b]carbazole**

Ce composé est préparé selon le même protocole que celui décrit dans l'exemple 1, à l'exception des stades D et F et de la modification suivante au stade E :

Stade E : Le temps de chauffe est ramené à 8 heures.

Rendement : 88 %
Point de fusion : 215°C

| Analyse élémentaire : $C_{22}H_{24}N_4O_2$, 0,5$H_2O$ | | | |
|---|---|---|---|
| | C % | H % | N % |
| calculé | 68,57 | 6,49 | 14,54 |
| trouvé | 68,64 | 6,53 | 14,48 |

**Exemple 9: 1-[2-(Pyrrolidin-1-yl)éthylaminocarbonyl)]-5-méthyl-9-méthoxy- 6*H*-pyrido[4,3-b]carbazole**

Ce composé est préparé selon le même protocole que celui décrit dans l'exemple 1, à l'exception des stades D et F qui sont supprimés et de l'amine utilisée au stade E qui est la 2-(pyrrolidin-1-yl)-1-aminoéthane.

**Exemple 10: 1-[2-(Pyrrolidin-1-yl)éthylaminocarbonyl]-5-méthyl-9-hydroxy-6*H*-pyrido[4,3-b]carbazole**

Ce composé est préparé selon le même protocole que celui décrit dans l'exemple 6 en utilisant au stade E la 2-(pyrrolidin-1-yl)-1-aminoéthane.

**Exemple 11: 1-{2-[(2-Hydroxy-éthyl)amino]éthylaminocarbonyl}-5,6-diméthyl-9-hydroxy-6*H*-pyrido[4,3-b]carbazole**

Stade A : 1-(5,6-Diméthyl-9-hydroxy-6*H*-pyrido[4,3-b]carbazolyl)carboxylate d'éthyle

L'ester obtenu à l'étape D de l'exemple 1 est engagée dans la réaction de déméthylation décrite à l'étape F de l'exemple 1, de manière à obtenir le 1-éthoxycarbonyl-5,6-diméthyl-9-hydroxy-6*H*-pyrido[4,3-b]carbazole.

Stade B : 1-{2-[(2-Hydroxy-éthyl)amino]éthylaminocarbonyl}-5,6-diméthyl-9-hydroxy-6*H*-pyrido[4,3-b]carbazole

0,4 g (1,198 mmoles) de l'ester obtenu à l'étape précédente sont dissous dans 10 ml de 2-(2-aminoéthylamino)éthanol sous atmosphère d'argon. Le mélange est porté à 120°C pendant 24 heures.

L'excès d'amine n'ayant pas réagi est éliminé par mise sous vide, le milieu réactionnel est ensuite additionné d'eau et de dichlorométhane. Le traitement habituel des phases organiques fournit un résidu huileux qui, repris à l'éthanol, est alors versé sur une solution d'éthanol saturée en acide chlorhydrique gazeux. L'évaporation de l'éthanol provoque la cristallisation de 0,58 g du dichlorhydrate dihydraté du produit attendu sous forme de cristaux rouges.

Rendement : 97 %

| Analyse élémentaire : $C_{22}H_{24}O_3N_4$, 2HCl, $2H_2O$ | | | | |
|---|---|---|---|---|
| | C % | H % | N % | Cl % |
| calculé | 52,70 | 6,03 | 11,17 | 14,14 |
| trouvé | 52,55 | 5,79 | 10,98 | 17,37 |

**Exemple 12 : 1-[(4-Aminobutyl)aminocarbonyl]-5,6-diméthyl-9-hydroxy-6*H*-pyrido[4,3-b]carbazole**

0,47 g (1,407 mmol) de l'ester obtenu à l'étape A de l'exemple 11 sont dissous dans 9 ml de 1,4-diaminobutane, sous atmosphère d'argon. Le mélange est chauffé à 130° pendant 3 heures.

L'excès d'amine n'ayant pas réagi est éliminé par mise sous vide. Le milieu réactionnel est ensuite additionné d'eau et de dichlorométhane.

La majeure partie du produit insoluble, est isolée par essorage et séchée. L'autre partie est obtenue par extraction au dichlorométhane qui est séché sur sulfate de magnésium, filtré puis évaporé à sec. L'ensemble du produit est recristallisé dans l'éthanol absolu pour donner 0,34 g de cristaux oranges correspondant au produit attendu.

Rendement : 62 %
Point de fusion : >95°C (décomposition)

| Analyse élémentaire : $C_{22}H_{24}N_4O_2$, $0,8H_2O$ | | | |
|---|---|---|---|
| | C % | H % | N % |
| calculé | 67,60 | 6,60 | 14,33 |
| trouvé | 67,85 | 6,98 | 13,02 |

**Exemple 13 : 1-[N-(2-Pyrrolidin-1-yléthyl)aminocarbonyl]-5,6-diméthyl-9-hydroxy-6*H*-pyrido[4,3-b]carbazole**

0,4 g (1,198 mmol) de l'ester obtenu à l'étape A de l'exemple 11 sont dissous dans 7,5 ml de 1-(2-aminoéthyl)pyrrolidine sous atmosphère d'argon. Le mélange est chauffé à 120°C pendant 16 heures.

L'excès d'amine est éliminé par mise sous vide et le milieu réactionnel est hydrolysé. Le traitement habituel de la phase organique fournit 0,341 g de cristaux jaunes correspondant au produit attendu.

Rendement : 65 %
Point de fusion : >120°C (Décomposition)

| Analyse élémentaire : $C_{24}H_{26}N_4O_2$, $2H_2O$ | | | |
|---|---|---|---|
| | C % | H % | N % |
| calculé | 66,03 | 6,97 | 12,84 |
| trouvé | 65,99 | 7,11 | 12,27 |

**Exemple 14 : 1-[N-(3-Morpholinopropyl)aminocarbonyl]-5,6-diméthyl-9-hydroxy-6*H*-pyrido[4,3-b]carbazole**

0,36 g (1,078 mmol) de l'ester obtenu à l'étape A de l'exemple 11 sont dissous dans 10 ml de 4-(3-aminopropyl)morpholine sous atmosphère d'argon. Le mélange est chauffé à 120°C pendant 19 heures.

L'excès d'amine n'ayant pas réagi est éliminé par mise sous vide. Le milieu réactionnel est hydrolysé et repris au dichlorométhane. Le mélange est laissé sous agitation pendant environ 1 heure. La majeure partie du produit insoluble, est isolée par essorage et séchée. L'autre partie est extraite au dichlorométhane qui est séché sur sulfate de magnésium, filtré et évaporé à sec. L'ensemble du produit est recristallisé dans l'éthanol absolu pour donner 0,42 g de cristaux jaunes correspondant au produit attendu.

Rendement : 90 %
Point de fusion : 110°C

| Analyse élémentaire : $C_{25}H_{28}N_4O_3$ | | | |
|---|---|---|---|
| | C % | H % | N % |
| calculé | 69,42 | 6,53 | 12,95 |
| trouvé | 69,34 | 6,50 | 12,86 |

**Exemple 15 : 1-[(N,N-Diméthyl-2-aminoéthyl)aminocarbonyl]-5-méthyl-6-éthyl-9-hydroxy-6*H*-pyrido[4,3-b]carbazole**

Stade A : 1-Ethoxycarbonyl-9-méthoxy-5-méthyl-6-éthyl-6H-pyrido[4,3-b] carbazole

A 5 g (15 mmol) de l'ester obtenu au stade C de l'exemple 1, dissous dans 10 ml de diméthylformamide, sont ajoutés 142 ml de carbonate de diéthyle, 3,75 g de carbonate de potassium ainsi que 200 mg d'éther couronne 18C6, sous atmosphère d'argon. Le mélange est chauffé à 130-140°C pendant 6 jours, puis concentré. Après addition d'eau et extraction au dichlorométhane le traitement habituel de la phase organique fournit 4,11 g de cristaux jaunes. (solvant de recristallisation : toluène).

Rendement : 76 %
Point de fusion : 149°C

| Analyse élémentaire : $C_{22}H_{22}N_2O_3$ | | | |
|---|---|---|---|
| | C % | H % | N % |
| calculé | 72,91 | 6,12 | 7,73 |
| trouvé | 73,06 | 6,19 | 7,72 |

Stade B : 1-Ethoxycarbonyl-9-hydroxy-5-méthyl-6-éthyl-6*H*-pyrido[4,3-b]carbazole

4 g (11,05 mmol) de l'ester obtenu à l'étape précédente sont dissous dans 440 ml de dichlorométhane sec, sous argon, à -78°C. 120 ml (110,5 mmol) de tribromure de bore (solution 1M dans du dichlorométhane) sont ajoutés. Le mélange est agité 2 heures 30 minutes à -78°C puis 1 heure à température ambiante. L'excès de tribromure de bore est hydrolysé par un goutte à goutte d'eau, en maintenant la température aux environs de 0°C. La solution est maintenue 1 heure sous agitation après alcalinisation par une solution ammoniacale. Le précipité formé est isolé et séché et les phases organiques traitées de manière habituelle. L'ensemble du produit brut est recristallisé dans le toluène pour fournir 2,98 g du composé attendu.

Rendement : 78 %
Point de fusion : 260°C

| Analyse élémentaire : $C_{21}H_{20}N_2O_3$ | | | |
|---|---|---|---|
| | C % | H % | N % |
| calculé | 72,40 | 5,79 | 8,04 |
| trouvé | 72,94 | 5,99 | 7,93 |

Stade C : 1-[(N,N-Diméthyl-2-aminoéthyl)aminocarbonyl]-5-méthyl-6-éthyl-9-hydroxy-6*H*-pyrido[4,3-b]carbazole

Le mélange formé par 0,8 g (2,30 mmol) d'ester obtenu à l'étape précédente dans 12 ml de N,N-diméthylaminoéthylènediamine sous atmosphère d'argon est chauffé à 115°C pendant 36 heures.Le milieu est alors hydrolysé puis extrait au dichlorométhane et la phase organique est séchée sur sulfate de magnésium puis filtrée.

Après évaporation du dichlorométhane, le résidu huileux est purifié par chromatographie sur colonne de gel de silice (éluant dichlorométhane/éthanol 9:1 et environ 0,5 % de triéthylamine). On obtient 0,424 g de cristaux jaunes correspondant au produit attendu.

Rendement : 44 %
Point de fusion : >115°C (Décomposition)

| Analyse élémentaire : $C_{23}H_{26}N_4O_2$, 1,5$H_2O$ | | | |
|---|---|---|---|
| | C % | H % | N % |
| calculé | 66,17 | 7,00 | 13,42 |
| trouvé | 66,01 | 6,81 | 13,41 |

**Etude pharmacologique**

Les exemples suivants permettent de mettre en évidence que l'index thérapeutique des meilleurs composés de l'invention est excellent :

- ils sont cytotoxiques,
- ils sont actifs à partir de doses inférieures à 5 mg/kg et jusqu' à 30 à 60 mg/kg,
- ils donnent de nombreux survivants aux doses optimales.

**Exemple A : Cytotoxicité des composés et des produits de référence**

Sept lignées cellulaires ont été utilisées :

- 2 leucémies murines, L1210 et P388 ;
- 1 carcinome épidermoïde humain, KB-3-1, et un carcinome pulmonaire humain S1 ;

- les lignées résistantes correspondantes, KB-A1 dont la résistance multidrogue a été induite par l'adriamycine (ADR) sur KB-3-1, P388/VCR-20 dont la résistance multidrogue a été induite par la vincristine (VCR) sur P388 et S1/tMDR dont la résistance multidrogue a été obtenue en transfectant le gène MDR1 humain dans les cellules S1.

Les cellules sont cultivées dans du milieu de culture RPMI 1640 complet contenant 10 % de sérum de veau foetal, 2 mM de glutamine, 50 unités/ml de pénicilline, 50 μg/ml de streptomycine et 10 mM d'Hepes.

Les cellules sont réparties dans des microplaques et exposées aux composés cytotoxiques. Les cellules sont ensuite incubées pendant environ 4 temps de doublement, soit 2 jours (P388, P388/VCR-20 et L1210) et 4 jours (KB-A1, KB-3-1, S1 et S1/tMDR). Le nombre de cellules viables est ensuite quantifié par un essai colorimétrique, le Microculture Tetrazolium Assay (Carmichael J., DeGraff W.G., Gazdar A.F., Minna J.D. and Mitchell J.R., Evaluation of a tetrazolium-based semiautomated colorimetric assay : assessment of chemosensitivity testing, Cancer Res., 47, 936-942, 1987). Les résultats sont exprimés en $IC_{50}$, concentration en cytotoxique qui inhibe à 50 % la prolifération des cellules tumorales. Les résultats obtenus sur la lignée L1210 sont rassemblés dans le tableau 1 :

| Produits | Cytotoxicité $IC_{50}$, nM |
|---|---|
| ex 1 | 2,6 |
| ex 2 | 4,3 |
| ex 3 | 574,3 |
| ex 4 | 874,7 |
| ex 5 | 1495,0 |
| ex 6 | 3,1 |
| ex 7 | 41,0 |
| ex 8 | 487,0 |
| ex 9 | 1495,5 |
| ex 11 | 29,7 |
| ex 12 | 449,8 |
| ex 13 | 13,1 |
| ex 14 | 10,4 |
| ADR | 26,4 |
| Acétate d'elliptinium | 138,9 |

Tableau 1 : Activité in vitro - Lignée L1210

Les résultats montrent que les produits des exemples 1, 2 et 6 sont environ 9 fois plus cytotoxiques que l'adriamycine et 46 fois plus que l'acétate d'elliptinium.

**Exemple B : Cytotoxicité des composés et des produits de référence sur des lignées présentant le phénotype de résistance multidrogue (MDR)**

Le facteur de résistance est défini par le rapport :

$$\frac{IC_{50} \text{ lignée résisistante}}{IC_{50} \text{ lignée sensible}}$$

| Lignées | Cytotoxicité, $IC_{50}$ (nM) et facteur de résistance | | | | |
|---|---|---|---|---|---|
| | **ex 1** | **ex 2** | **ex 6** | **ADR** | **Acétate d'elliptinium** |
| sensible P388 | 5 | 11 | 19 | 20 | - |
| résistante P388/VCR-20 | 4 | 38 | 10 | 340 | - |
| facteur de résistance | 0,8 | 3,4 | 0,5 | 17 | - |
| sensible KB-3-1 | 12 | 19 | 20 | 5 | 226 |
| résistante KB-A1 | 120 | 1217 | 737 | 6869 | 4322 |
| facteur de résistance | 10 | 64 | 37 | 1374 | 19 |
| sensible S1 | 49 | 57 | 42 | 36 | - |
| résistante S1/tMDR | 25 | 38 | 29 | 135 | - |
| facteur de résistance | 0,5 | 0,7 | 0,7 | 3,8 | - |

Tableau 2 : Cytotoxicités et facteurs de résistance

La résistance croisée des lignées résistantes aux dérivés est beaucoup plus faible que la résistance à l'adriamycine. L'activité des composés est très supérieure à celle de l'adriamycine ou de l'acétate d'elliptinium sur KB-A1. Les lignées résistantes P388/VCR-20 et S1/tMDR (dont la résistance à l'ADR est plus faible) sont plus sensibles aux exemples 1 et 6 que les lignées sensibles correspondantes. Ces dérivés pourraient donc être utilisés contre des tumeurs résistantes à l'adriamycine.

**Exemple C : Activité in vivo.**

**Exemple C-1 : Activité antitumorale des composés sur la leucémie P388**

La lignée P388 (leucémie murine) a été fournie par le National Cancer Institute (NCI) (Frederick, USA). Les cellules tumorales ($10^6$ cellules) ont été inoculées au jour 0 dans la cavité péritonéale de souris BDF1 femelles (Iffa-Credo, France). Huit à dix souris, dont le poids varie entre 18 et 20 g, sont utilisées par groupe expérimental.

Les produits ont été administrés au jour 1, aux doses indiquées par voie intrapéritonéale.

L'activité antitumorale est exprimée en % de T/C :

$$\% \text{ T/C} = \frac{\text{Temps de survie médian des animaux traités}}{\text{Temps de survie médian des animaux contrôles}} \times 100$$

Les animaux survivants à 60 jours (survivants à long terme) sont indiqués.

Les résultats sont indiqués sur le tableau 3 suivant :

| Produits | Dose (mg/kg) | T/C (%) | Survivants à J60/nbre total de souris |
|---|---|---|---|
| exemple 1 | 5<br>10<br>20<br>30<br>60 | 163<br>183<br>220<br>200<br>257 | 1/10<br>1/10<br>2/10<br>3/10<br>3/10 |
| exemple 2 | 5<br>10<br>20<br>30<br>40<br>50<br>60 | 177<br>190<br>250<br>204<br>219<br>222<br>248 | 0/10<br>0/10<br>3/10<br>1/10<br>2/10<br>3/10<br>3/10 |
| exemple 6 | 5<br>10<br>20<br>30 | 280<br>233<br>> 600<br>> 600 | 4/10<br>3/10<br>5/10<br>7/10 |
| ADR | 10 | 290 | 2/10 |
| Acétate d'elliptinium | 5 | 171 | 0/10 |

Tableau 3 : Activité in vivo : leucémie P388

Les composés ont été testés à des doses équitoxiques. La variation pondérale, à la dose optimale utilisée pour les dérivés est de -0,4 à -0,7 g alors qu'elle est de -0,7 g pour l'adriamycine et de -4 g pour l'acétate d'elliptinium.

Les composés sont actifs dès 5 mg/kg, toutes les doses utilisées donnant des survivants à long terme pour deux dérivés (exemples 1 et 6 du tableau 2). Le produit de l'exemple 1 utilisé en une seule administration à 30 mg/kg guérit 7 souris sur 10 traitées alors que l'adriamycine n'en guérit que 2 et l'acétate d'elliptinium aucune. Les trois dérivés testés sont considérablement plus actifs que l'acétate d'elliptinium qui de plus est toxique à partir de 5 mg/kg.

Exemple C-2 : Activité antitumorale des composés sur le carcinome pulmonaire de Lewis

Le carcinome pulmonaire de Lewis (fourni par le NCI, Frederick, USA) a été greffé sous forme de fragments à JO et par voie sous-cutanée à des souris B6D2F1 femelles pesant de 18 à 20 g. Les produits ont été administrés aus doses indiquées par voie i.v. à J3, J6 et J9.

L'activité antitumorale a été déterminée à J20 par la mesure du volume tumoral :

$$T/C\ \%\ (\text{volume}) = \frac{\text{volume tumoral médian des animaux traités}}{\text{volume tumoral médian des animaux témoins}} \times 100$$

et par la prolongation de la survie des groupes traités, exprimée en T/C %. Les survivants à long terme (J90) sont notés.

$$T/C\ \%\ (\text{survie}) = \frac{\text{temps médian de survie des animaux traités}}{\text{temps médian de survie des animaux témoins}} \times 100$$

Le composé de l'exemple 1 est très actif sur ce modèle très résistant. La dose de 40 mg/kg est curative pour 100 % des animaux alors que l'adriamycine, à la dose maximale tolérée, est beaucoup moins active (tableau 4).

| Produit | Dose (mg/kg) | Voie | T/C médian, % (volume) J20 | T/C médian, % (survie) | Survivants à J90 |
|---|---|---|---|---|---|
| Témoin | - | i.v. | 100 | 100 | 1/25 |
| ex. 1 | 20<br>40<br>60 | i.v.<br>i.v.<br>i.v. | 1<br>0<br>0 | > 300<br>> 300<br>> 300 | 6/10<br>10/10<br>6/10 |
| ADR | 2,5<br>5<br>10 | i.v.<br>i.v.<br>i.v. | 63<br>44<br>1 | 108<br>125<br>128 | 0/10<br>2/10<br>3/10 |

Tableau 4 : Activité antitumorale des produits sur le carcinome de Lewis

Exemple C-3 : Activité antitumorale des composés sur le sarcome M5076

Le sarcome M5076 est fourni par le NCI (Frederick, USA). Les cellules tumorales ($10^7$ cell./animal) ont été Inoculées à J0 dans la cavité péritonéale de souris femelles B6D2F1. Les produits ont été administrés par voie i.p. à J1, J5, J9 et J13 aux doses indiquées. La survie des animaux a été notée à J90 et le T/C survie (%) a été calculé.
Le composé de l'exemple 1, à la dose de 20 mg/kg, est très actif sur cette tumeur. On observe 6 survivants sur 10 à J90. L'acétate d'elliptinium est peu actif à la dose maximale tolérée (2 mg/kg), aucun animal n'a survécu. Les résultats sont reproduits dans le tableau 5.

| Produit | Dose (mg/kg) | Voie | T/C médian, % (survie) | Survivants à J90 |
|---|---|---|---|---|
| Témoin | - | i.p. | 100 | 0/32 |
| ex. 1 | 5<br>10<br>20 | i.p.<br>i.p.<br>i.p. | 168<br>301<br>> 352 | 0/10<br>4/10<br>6/10 |
| ADR | 5 | i.p. | 320 | 4/10 |
| Ac. ellipt. | 1<br>2 | i.p.<br>i.p. | 153<br>180 | 1/10<br>0/10 |

Tableau 5 : Activité antitumorale des produits sur le sarcome M5076

Exemple C-4 : Activité antitumorale des composés sur le côlon 38

Le côlon 38 (fourni par le NCI, Frederick, USA) a été greffé sous forme de fragments par voie sous-cutané a des souris B6D2F1 femelles. Les produits ont été administrés par voie i.v. à J2 et J9 et l'activité antitumorale a été déterminée à J25 par mesure du volume tumoral (T/C volume, %).
Le composé de l'exemple 2, administré par voie i.v. est très actif sur cette tumeur solide dès la dose de 20 mg/kg (T/C = 0 %) (tableau 6). Il est autant actif que le 5-fluoro-uracyle (5-Fu), utilisé à la dose de 80 mg/kg.

| Produit | Dose (mg/kg) | Voie | T/C médian, % (volume tumoral) |
|---|---|---|---|
| ex. 1 | 20<br>40<br>60 | i.v.<br>i.v.<br>i.v. | 41<br>16<br>0 |
| ex. 2 | 20<br>40<br>60 | i.v.<br>i.v.<br>i.v. | 0<br>0<br>0 |
| ex. 6 | 10<br>20<br>30 | i.v.<br>i.v.<br>i.v. | 35<br>22<br>0 |
| ADR | 15 | i.v. | 0 |
| 5-Fu | 80 | i.v. | 0 |

Tableau 6 : Activité antitumorale des produits sur le côlon 38

## Revendications

1. Composés de formule générale (I) :

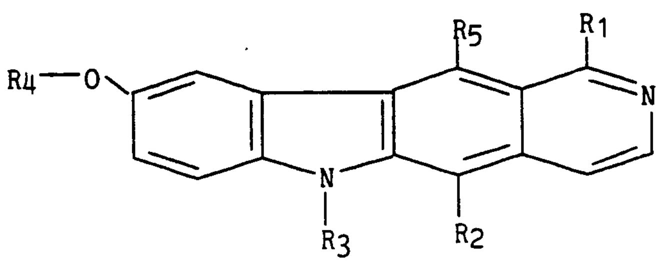

(I)

dans laquelle :

$R_1$ représente le groupement

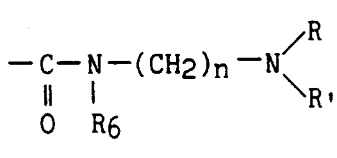

dans lequel :

- n est un entier compris entre 1 et 6,
- R, R' et $R_6$ identiques ou différents sont choisis indépendamment les uns des autres parmi l'hydrogène et un radical alkyle linéaire ou ramifié contenant 1 à 6 atomes de carbone, éventuellement substitués par un

ou plusieurs groupements hydroxy,

- ou bien R et R' forment ensemble avec l'atome d'azote auquel ils sont rattachés un hétérocycle contenant éventuellement un second hétéroatome, lequel hétérocycle peut être substitué par un ou plusieurs radicaux alkyles linéaires ou ramifiés contenant 1 à 6 atomes de carbone, et $R_6$ est tel que défini précédemment,
- ou bien R et $R_6$ forment ensemble un hétérocycle substitué ou non par un ou plusieurs radicaux alkyles linéaires ou ramifiés contenant 1 à 6 atomes de carbone, et R' est tel que défini précédemment,
- $R_2$ représente un radical alkyle linéaire ou ramifié contenant 1 à 6 atomes de carbone,
- $R_3$ et $R_4$ et $R_5$ identiques ou différents sont choisis indépendamment les uns des autres parmi l'hydrogène et un radical alkyle linéaire ou ramifié contenant 1 à 6 atomes de carbone,

leurs isomères optiques ainsi que leurs éventuels N-oxydes et sels d'addition à un acide ou à une base, pharmaceutiquement acceptable.

**2.** Composé selon la revendication 1 qui est le 1-(N,N-diméthylaminoéthyl-aminocarbonyl)-5,6-diméthyl-9-hydroxy-6*H*-pyrido[4,3-b]carbazole,
ses N-oxydes ainsi que ses sels d'addition à un acide, pharmaceutiquement acceptables.

**3.** Composé selon la revendication 1 qui est le 1-(N,N-diméthylaminopropylaminocarbonyl)-5,6-diméthyl-9-hydroxy-6*H*-pyrido[4,3-b]carbazole,
ses N-oxydes ainsi que ses sels d'addition à un acide, pharmaceutiquement acceptables.

**4.** Composé selon la revendication 1 qui est le 1-(N,N-diméthylaminoéthylaminocarbonyl)-5-méthyl-9-hydroxy-6*H*-pyrido[4,3-b]carbazole.
ses N-oxydes ainsi que ses sels d'addition à un acide, pharmaceutiquement acceptables.

**5.** Composé selon la revendication 1 qui est le 1-[N-(3-morpholinopropyl)aminocarbonyl]-5,6-diméthyl-9-hydroxy-6*H*-pyrido[4,3-b] carbazole,
ses N-oxydes ainsi que ses sels d'addition à un acide, pharmaceutiquement acceptables.

**6.** Composé selon la revendication 1 qui est le 1-[N-(2-pyrrolidin-1-yl-éthyl)aminocarbonyl]-5,6-diméthyl-9-hydroxy-6*H*-pyrido[4,3-b]carbazole,
ses N-oxydes ainsi que ses sels d'addition à un acide, pharmaceutiquement acceptables.

**7.** Procédé de préparation des composés de formule (I) caractérisé par une première réaction dans laquelle on fait réagir dans l'acide acétique un composé de formule (II) avec un composé de formule (III):

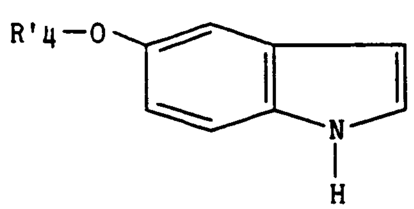

(II)

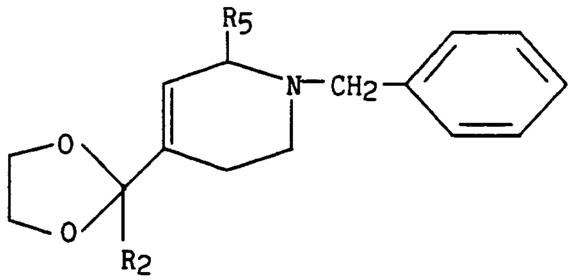

(III)

dans lesquelles $R_2$ et $R_5$ possèdent les mêmes significations que dans la formule (I) et $R'_4$ représente un radical alkyle linéaire ou ramifié contenant 1 à 6 atomes de carbone,
pour obtenir, après débenzylation puis chauffage en présence de dialkyloxalate un composé de formule (IV) :

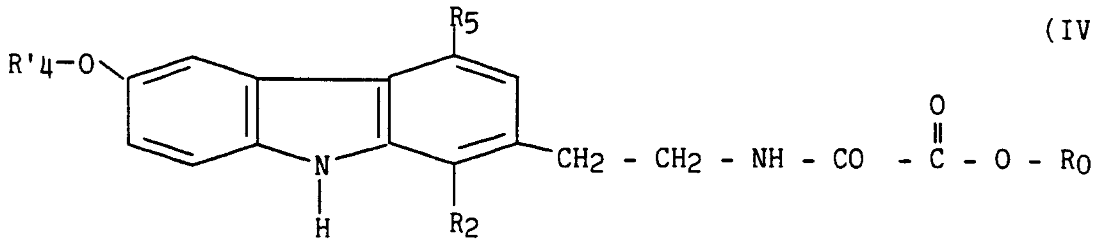

(IV)

dans laquelle $R_2$, $R'_4$ et $R_5$ possèdent les mêmes significations que précédemment et $R_0$ représente un radical alkyle contenant de 1 à 5 atomes de carbone,
dont un chauffage à reflux en présence de $POCl_3$ dans le toluène permet la cyclisation, pour conduire après déshydrogénation sur charbon au palladium au composé de formule (Va)

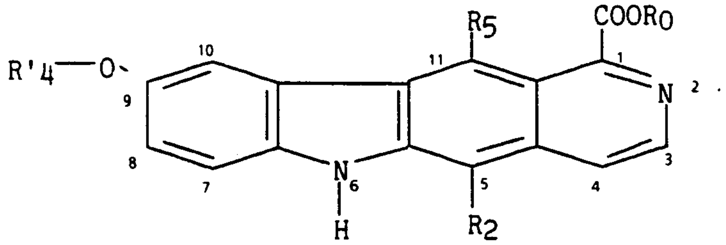

(Va)

dans laquelle $R_0$,$R_2$, $R'_4$ et $R_5$ ont les mêmes significations que précédemment,
dont on substitue, si on le désire, l'azote 6 du carbazole par traitement avec un carbonate de dialkyle, de formule

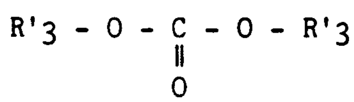

dans laquelle $R'_3$ représente un radical alkyle linéaire ou ramifié contenant 1 à 6 atomes de carbone,
dans un solvant polaire, en présence d'un carbonate alcalin, pour obtenir un composé de formule (Vb) :

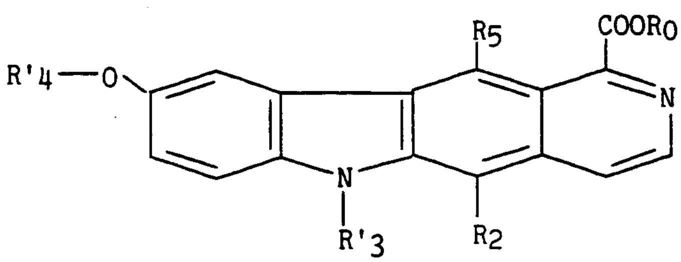

(Vb)

dans laquelle $R_2$, $R'_3$, $R'_4$, $R_5$ et $R_0$ possèdent les mêmes significations que précédemment,
composés de formule (Va) et (Vb) formant l'ensemble des composés de formule (V) :

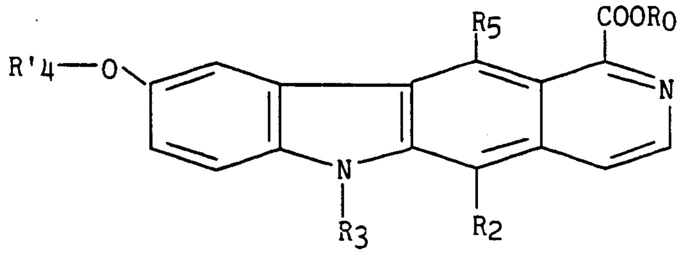

(V)

dans laquelle $R_2$, $R_3$, $R'_4$, $R_5$ et $R_0$ possèdent les mêmes significations que précédemment,
composés de formule (V) que l'on soumet à une réaction de substitution par un composé de formule (VI) :

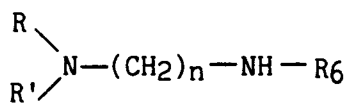

(VI)

dans laquelle R, R', $R_6$ et n possèdent les mêmes significations que dans la formule (I),
pour obtenir les composés de formule (Ia) :

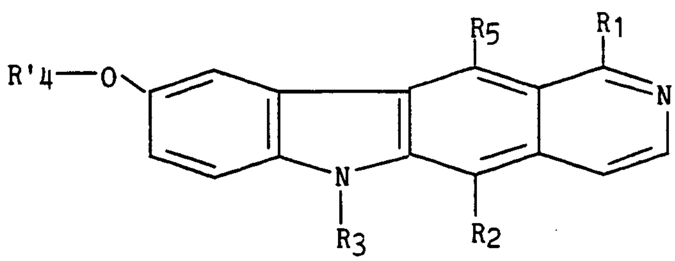

(Ia)

dans laquelle $R_1$, $R_2$, $R_3$, $R'_4$ et $R_5$ possèdent les mêmes significations que précédemment,
composés de formule (Ia) que l'on peut déalkyler par ajout d'un trihalogénure de bore, pour donner les composés de formule (Ib) :

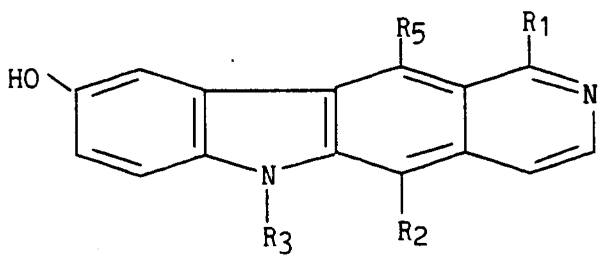

(Ib)

dans laquelle $R_1$, $R_2$, $R_3$ et $R_5$ ont les mêmes significations que précédemment,
composés de formule (Ia) et (Ib) formant l'ensemble des composés de formule générale (I) que l'on purifie le cas échéant selon une technique classique de séparation et que l'on transforme, si nécessaire, en leurs éventuels N-oxydes et en leurs sels d'addition à un acide ou une base, pharmaceutiquement acceptables.

**8.** Compositions pharmaceutiques contenant comme principe actif au moins un composé selon l'une quelconque des revendications 1 à 6, seul ou en combinaison avec un ou plusieurs excipients ou véhicule inertes, non toxiques, pharmaceutiquement acceptables.

**9.** Compositions pharmaceutiques selon la revendication 8 contenant au moins un principe actif, agent antitumoral inhibiteur de topoisomérases, selon l'une quelconque des revendications 1 à 6, utiles dans le traitement des cancers.

## Claims

**1.** Compounds of the general formula (I) :

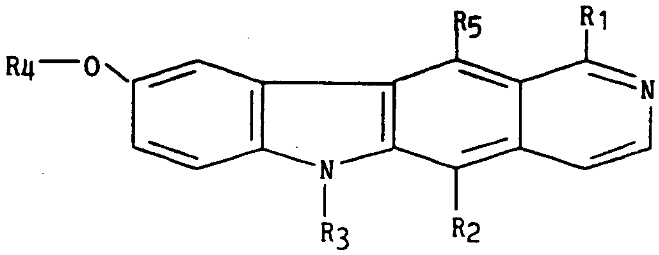

(I)

wherein :

$R_1$ represents the group

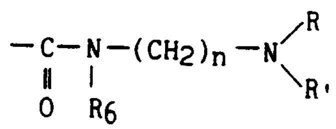

in which :

- n is an integer of from 1 to 6,
- R, R' and $R_6$, which are the same or different, are selected, independently of one another, from hydrogen and a straight-chain or branched alkyl radical containing from 1 to 6 carbon atoms optionally substituted by one or more hydroxy groups,
- or R and R', together with the nitrogen atom to which they are attached, form a heterocycle optionally containing a second hetero atom, which heterocycle may be substituted by one or more straight-chain or branched alkyl radicals containing from 1 to 6 carbon atoms, and $R_6$ is as defined above,
- or R and $R_6$ together form a heterocycle which is unsubstituted or substituted by one or more straight-chain or branched alkyl radicals containing from 1 to 6 carbon atoms, and R' is as defined above,
- $R_2$ represents a straight-chain or branched alkyl radical containing from 1 to 6 carbon atoms,
- $R_3$, $R_4$ and $R_5$, which are the same or different, are selected, independently of one another, from hydrogen and a straight-chain or branched alkyl radical containing from 1 to 6 carbon atoms,

their optical isomers and also their possible N-oxides and pharmaceutically acceptable addition salts with an acid or base.

**2.** Compound according to claim 1 which is 1-(N,N-di-methylaminoethylaminocarbonyl)-5,6-dimethyl-9-hydroxy-6H-pyrido[4,3-b]carbazole, its N-oxides and also the pharmaceutically acceptable addition salts thereof with an acid.

**3.** Compound according to claim 1 which is 1-(N,N-di-methylaminopropylaminocarbonyl)-5,6-dimethyl-9-hydroxy-6H-pyrido[4,3-b]carbazole, its N-oxides and also the pharmaceutically acceptable addition salts thereof with an acid.

**4.** Compound according to claim 1 which is 1-(N,N-di-methylaminoethylaminocarbonyl)-5-methyl-9-hydroxy-6H-pyrido[4,3-b]carbazole, its N-oxides and also the pharmaceutically acceptable addition salts thereof with an acid

5. Compound according to claim 1 which is 1-[N-(3-morpholinopropyl)aminocarbonyl]-5,6-dimethyl-9-hydroxy-6<u>H</u>-pyrido[4,3-b]carbazole, its N-oxides and also the pharmaceutically acceptable addition salts thereof with an acid.

6. Compound according to claim 1 which is 1-[N-(2-pyrrolidin-1-ylethyl)aminocarbonyl]-5,6-dimethyl-9-hydroxy-6<u>H</u>-pyrido[4,3-b]carbazole, its N-oxides and also the pharmaceutically acceptable addition salts thereof with an acid.

7. Process for the preparation of compounds of formula (I), characterised by a first reaction in which a compound of formula (II) is reacted in acetic acid with a compound of formula (III):

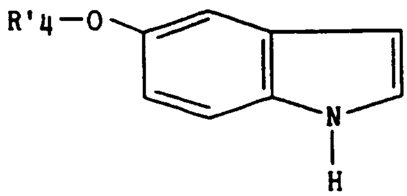

(II)

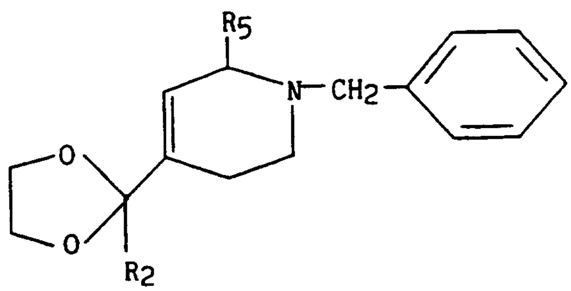

(III)

wherein $R_2$ and $R_5$ are as defined for formula (I) and $R'_4$ represents a straight-chain or branched alkyl radical containing from 1 to 6 carbon atoms,
to obtain, after debenzylation followed by heating in the presence of a dialkyl oxalate, a compound of formula (IV) :

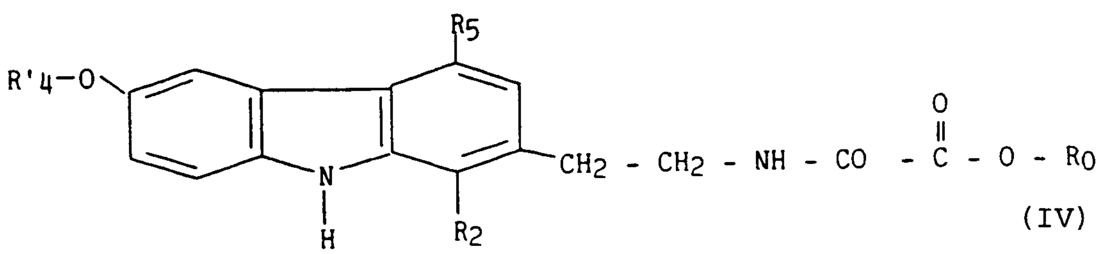

(IV)

wherein $R_2$, $R'_4$ and $R_5$ are as defined hereinbefore and $R_0$ represents an alkyl radical containing from 1 to 5 carbon atoms,
which may be cyclised by heating at reflux in the presence of $POCl_3$ in toluene so as to yield, after dehydrogenation on palladium-on-carbon, a compound of formula (Va) :

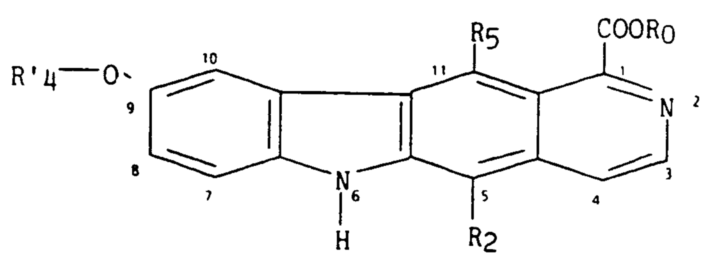

(Va)

wherein $R_0$, $R_2$, $R'_4$ and $R_5$ are as defined hereinbefore,

if desired, the 6-nitrogen atom of the carbazole of which is substituted by treatment with a dialkyl carbonate of formula

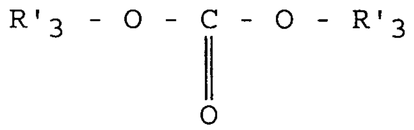

wherein $R'_3$ represents a straight-chain or branched alkyl radical containing from 1 to 6 carbon atoms, in a polar solvent, in the presence of an alkali metal carbonate, to obtain a compound of formula (Vb) :

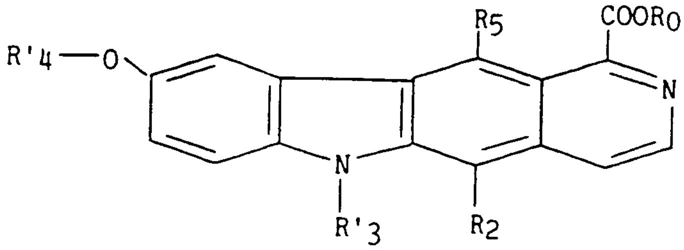

(Vb)

wherein $R_2$, $R'_3$, $R'_4$, $R_5$ and $R_0$ are as defined hereinbefore, the compounds of formulae (Va) and (Vb) forming the totality of the compounds of formula (V) :

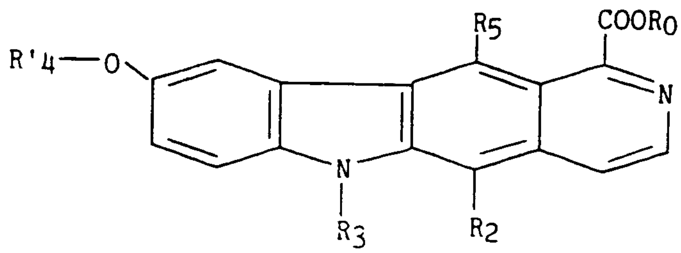

(V)

wherein $R_2$, $R_3$, $R'_4$, $R_5$ and $R_0$ are as defined hereinbefore, which compounds of formula (V) are subjected to a substitution reaction with a compound of formula (VI) :

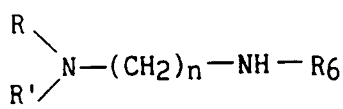

(VI)

wherein R, R', $R_6$ and n are as defined for formula (I), to obtain compounds of formula (Ia) :

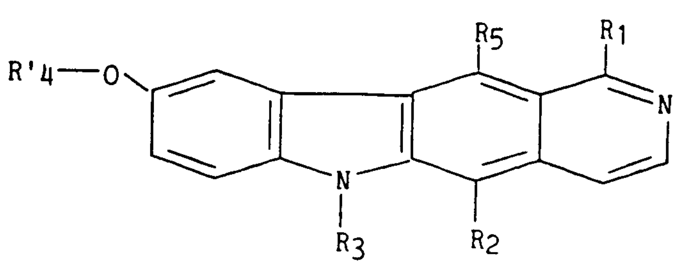

(Ia)

wherein $R_1$, $R_2$, $R_3$, $R'_4$ and $R_5$ are as defined hereinbefore, which compounds of formula (Ia) may be dealkylated by the addition of a boron trihalide to yield compounds of for-

mula (Ib) :

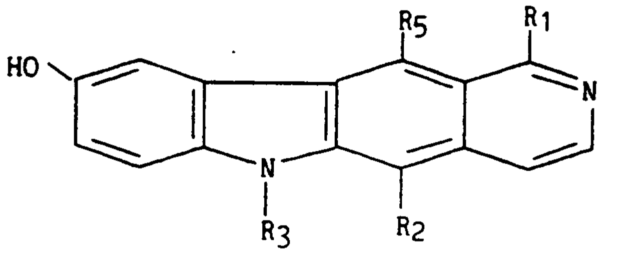

(Ib)

wherein $R_1$, $R_2$, $R_3$ and $R_5$ are as defined hereinbefore,

the compounds of formula (Ia) and (Ib) forming the totality of the compounds of the general formula (I) which, if necessary, are purified according to a conventional separation technique and, if necessary, are converted into their possible N-oxides and into their pharmaceutically acceptable addition salts with an acid or base.

**8.** Pharmaceutical compositions comprising as active ingredient at least one compound according to any one of claims 1 to 6, alone or in combination with one or more inert, non-toxic, pharmaceutically acceptable excipients or carriers.

**9.** Pharmaceutical compositions according to claim 8 comprising at least one active ingredient, an anti-tumour agent inhibiting topoisomerases, according to any one of claims 1 to 6, for use in the treatment of cancers.

**Patentansprüche**

**1.** Verbindungen der allgemeinen Formel (I):

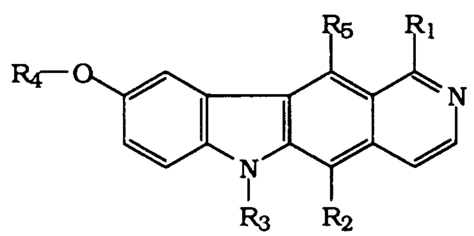

(I)

in der:

$R_1$ die Gruppe

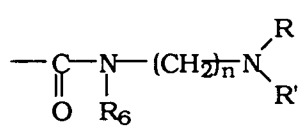

in der:

- n eine ganze Zahl zwischen 1 und 6 darstellt.
- R, R' und $R_6$ die gleichartig oder verschieden sind, unabhängig voneinander aus Wasserstoff und geradkettigen oder verzweigten Alkylgruppen mit 1 bis 6 Kohlenstoffatomen, die gegebenenfalls durch eine oder mehrere Hydroxylgruppen substituiert sein können, ausgewählt sind.
- oder R und R' gemeinsam mit dem Stickstoffatom, an das sie gebunden sind, einen Heterocyclus bilden, der gegebenenfalls ein zweites Heteroatom enthält, welcher Heterocyclus durch eine oder mehrere geradkettige oder verzweigte Alkylgruppen mit 1 bis 6 Kohlenstoffatomen substituiert ist und $R_6$ die oben ange-

gebenen Bedeutungen besitzt,

- oder R und $R_6$ gemeinsam einen Heterocyclus bilden, der gegebenenfalls durch eine oder mehrere geradkettige oder verzweigte Alkylgruppen mit 1 bis 6 Kohlenstoffatomen substituiert ist, und R' die oben angegebenen Bedeutungen besitzt,
- $R_2$ eine geradkettige oder verzweigte Alkylgruppe mit 1 bis 6 Kohlenstoffatomen darstellt, und
- $R_3$ und $R_4$ und $R_5$, die gleichartig oder verschieden sind, unabhängig voneinander aus Wasserstoff und geradkettigen oder verzweigten Alkylgruppen mit 1 bis 6 Kohlenstoffatomen ausgewählt sind,

deren optische Isomere sowie deren mögliche N-Oxide und deren Additionssalze mit einer pharmazeutisch annehmbaren Säure oder Base.

2. Verbindung nach Anspruch 1, nämlich 1-(N,N-Dimethylaminoethyl-aminocarbonyl)-5,6-dimethyl-9-hydroxy-6*H*-pyrido[4,3-b]carbazol, dessen N-Oxide sowie dessen Additionssalze mit einer pharmazeutisch annehmbaren Säure.

3. Verbindung nach Anspruch 1, nämlich 1-(N,N-Dimethylaminopropyl-aminocarbonyl)-5,6-dimethyl-9-hydroxy-6*H*-pyrido[4,3-b]carbazol, dessen N-Oxide sowie dessen Additionssalze mit einer pharmazeutisch annehmbaren Säure.

4. Verbindung nach Anspruch 1, nämlich 1-(N,N-Dimethylaminoethyl-aminocarbonyl)-5-methyl-9-hydroxy-6*H*-pyrido[4,3-b]carbazol, dessen N-Oxide sowie dessen Additionssalze mit einer pharmazeutisch annehmbaren Säure.

5. Verbindung nach Anspruch 1, nämlich 1-[N-(3-Morpholinopropyl)-aminocarbonyl]-5,6-dimethyl-9-hydroxy-6*H*-pyrido[4,3-b]carbazol, dessen N-Oxide sowie dessen Additionssalze mit einer pharmazeutisch annehmbaren Säure.

6. Verbindung nach Anspruch 1, nämlich 1-[N-(2-Pyrrolidin-1-yl-ethyl)-aminocarbonyl)-5,6-dimethyl-9-hydroxy-6*H*-pyrido[4,3-b]carbazol, dessen N-Oxide sowie dessen Additionssalze mit einer pharmazeutisch annehmbaren Säure.

7. Verfahren zur Herstellung der Verbindungen der Formel (I), gekennzeichnet durch eine erste Reaktion, bei der man eine Verbindung der Formel (II) in Essigsäure mit einer Verbindung der Formel (III) umsetzt:

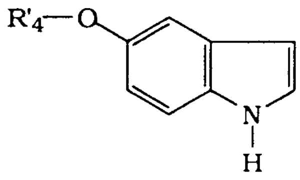

(II)

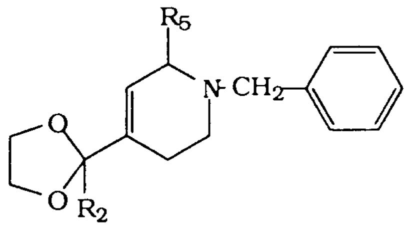

(III)

in denen $R_2$ und $R_5$ die bezüglich der Formel (I) angegebenen Bedeutungen besitzen und $R'_4$ eine geradkettige oder verzweigte Alkylgruppe mit 1 bis 6 Kohlenstoffatomen darstellt,
so daß man nach der Debenzylierung durch Erhitzen in Gegenwart von Dialkyloxalat eine Verbindung der Formel (IV) erhält:

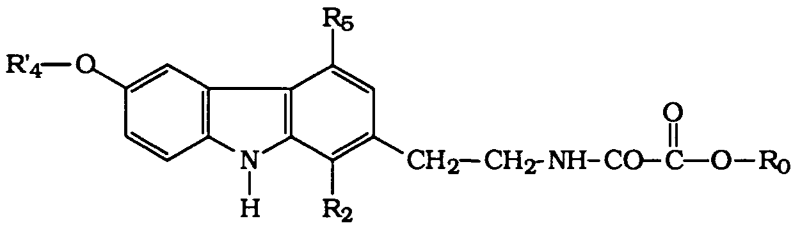

(IV)

in der $R_2$, $R'_4$ und $R_5$ die oben angegebenen Bedeutungen besitzen und $R_0$ eine Alkylgruppe mit 1 bis 5 Kohlenstoffatomen darstellt,
deren Erhitzen zum Sieden am Rückfluß in Gegenwart von $POCl_3$ in Toluol eine Cyclisierung ermöglicht, so daß man nach der Dehydrierung über Palladium-auf-Kohlenstoff die Verbindung der Formel (Va) erhält:

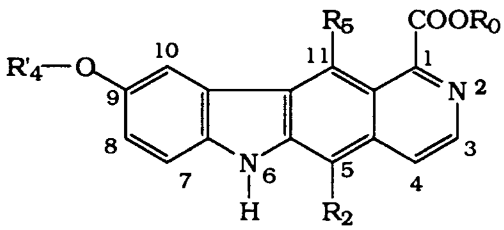

(Va)

in der $R_0$, $R_2$, $R'_4$ und $R_5$ die oben angegebenen Bedeutungen besitzen, bei der man gewünschtenfalls das Stickstoffatom in der 6-Stellung des Carbazols durch Behandeln mit einem Dialkylcarbonat der Formel

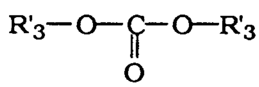

in der $R'_3$ eine geradkettige oder verzweigte Alkylgruppe mit 1 bis 6 Kohlenstoffatomen darstellt,
In einem polaren Lösungsmittel und in Gegenwart eines Alkalimetallcarbonats substituiert, so daß man eine Verbindung der Formel (Vb) erhält:

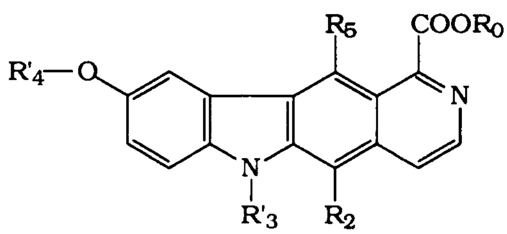

(Vb)

in der $R_2$, $R'_3$, $R'_4$, $R_5$ und $R_0$ die oben angegebenen Bedeutungen besitzen, welche Verbindungen der Formeln (Va) und (Vb) die Gesamtheit der Verbindungen der Formel (V) bilden:

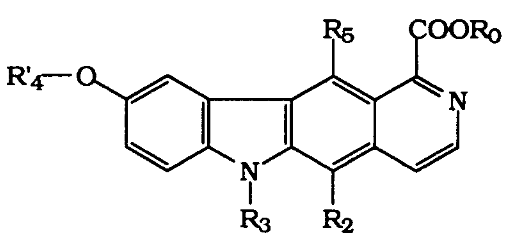

(V)

in der $R_2$, $R_3$, $R'_4$, $R_5$ und $R_0$ die oben angegebenen Bedeutungen besitzen, welche Verbindungen der Formel (V) man einer Substitutionsreaktion mit einer Verbindung der Formel (VI) unterwirft:

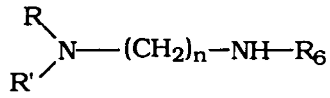

(VI)

In der R, R', $R_6$ und n die bezüglich der Formel (I) angegebenen Bedeutungen besitzen, zur Bildung der Verbindungen der Formel (Ia):

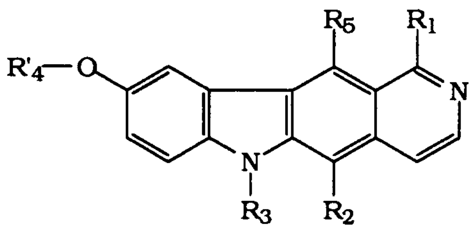

(Ia)

in der $R_1$, $R_2$, $R_3$, $R'_4$ und $R_5$ die oben angegebenen Bedeutungen besitzen, welche Verbindungen der Formel (Ia) man durch Zugabe eines Bortrihalogenids dealkylieren kann zur Bildung der Verbindungen der Formel (Ib):

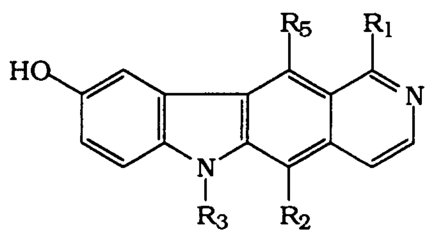

(Ib)

in der $R_1$, $R_2$, $R_3$ und $R_5$ die oben angegebenen Bedeutungen besitzen, welche Verbindungen der Formeln (Ia) und (Ib) die Gesamtheit der Verbindungen der allgemeinen Formel (I) bilden, welche man gegebenenfalls mit Hilfe einer klassischen Trennungsmethode reinigt und erforderlichenfalls in ihre möglichen N-Oxide und ihre Additionssalze mit pharmazeutisch annehmbaren Säuren oder Basen überführt.

**8.** Pharmazeutische Zubereitungen enthaltend als Wirkstoff mindestens eine Verbindung nach einem der Ansprüche 1 bis 6 allein oder in Kombination mit einem oder mehreren inerten, nichttoxischen, pharmazeutisch annehmbaren Trägermaterialien oder Bindemitteln.

**9.** Pharmazeutische Zubereitungen nach Anspruch 8 enthaltend als Wirkstoff mindestens einen antitumoral wirksamen Topoisomerase-Inhibitor nach einem der Ansprüche 1 bis 6 zur Behandlung von Krebs.